# EUROPÄISCHE PATENTANMELDUNG

(11) **EP 3 973 943 A1**
(43) Veröffentlichungstag der Anmeldung: **30.03.2022**
(21) Anmeldenummer: 20197916.8
(22) Anmeldetag: 23.09.2020
(51) Int. Cl.: A61K 8/22, A61K 8/34, A61K 8/92, A61K 8/9789, A61Q 11/00, A61Q 17/00, A61K 8/04

(54) **MUNDSPÜLUNG, INSBESONDERE ZUR BEHANDLUNG DES MENSCHLICHEN MUND- UND RACHENRAUMS**

(71) Anmelder: Gallmetzer, Dietrich, 39100 Bozen (IT)
(72) Erfinder: Gallmetzer, Dietrich, 39100 Bozen (IT)
(74) Vertreter: Tergau & Walkenhorst Patentanwälte PartGmbB

(57) **Zusammenfassung**

Eine Mundspülung soll im Hinblick auf den vorgesehenen Einsatz im menschlichen Mund- und Rachenraum bei hoher Verträglichkeit für den Menschen oder menschliches Gewebe und unter Ausschluss möglicher Resistenzenbildung eine im Vergleich zu bekannten Mundspülungen deutliche erhöhte keimtötende Wirkung aufweisen. Dazu ist zur Bildung der Mundspülung erfindungsgemäß erfindungsgemäß eine wässrige Zusammensetzung vorgesehen, die als Bestandteile zusätzlich zu Wasser eine Anzahl von Pflanzenextrakten und einen antimikrobiellen Wirkstoff aufweist. Vorzugsweise ist dabei als antimikrobieller Wirkstoff eine Kombination aus Alkohol und Wasserstoffperoxid (H₂O₂) vorgesehen.

## Beschreibung

Die Erfindung bezieht sich auf eine Mundspülung, insbesondere zur Behandlung des menschlichen Mund- und Rachenraums.

Mundspülungen, teilweise auch als Mundwasser oder Mundspül-Lösung bezeichnet, sind in vielfältiger Form und Ausgestaltung bekannt und gebräuchlich. Sie werden vielfach zur Zahnpflege oder zur Zahnerkrankungs-Prophylaxe angewandt, und sie bestehen meist aus einer Flüssigkeit, welche Bakterien und Keime im Mundraum dezimieren soll.

Bekannte Mundspülungen sind in der Regel ein Alkohol-Wasser-Gemisch und enthalten meist weitere Inhaltsstoffe. Der Alkohol, beispielsweise Ethanol oder Propandiol, kann hierbei der Desinfektion und/oder der Konservierung dienen. Ferner lösen sich viele Stoffe, welche häufig in Mundspülungen verwendet werden, deutlich besser in einem Alkohol-Wasser-Gemisch als in reinem Wasser, so dass der Alkohol auch als Lösungsvermittler dienen kann. So werden bekannten Mundspülungen beispielsweise antiseptische Wirkstoffe wie Chlorhexidin beigesetzt, welche sich teilweise deutlich besser in Alkohol-Wasser-Gemischen lösen als in reinem Wasser. Ferner enthalten einige bekannte Mundspülungen Fluoride, welche der Härtung des Zahnschmelzes dienen sollen.

Diese bekannten Mundspülungen sind hinsichtlich ihrer Zusammensetzung und Wirkungsweise üblicherweise vornehmlich auf einen Pflegeansatz ausgerichtet. Eine desinfizierende Wirkung, also insgesamt gesehen eine Reduktion der Keim- oder Bakterienlast, ist hierbei zwar insgesamt gesehen auch vorgesehen und erwünscht, spielt dennoch aber nur eine untergeordnete Rolle. Allerdings erfordern auch zahlreiche, insbesondere entzündliche, Pathologien aus der Mundhöhle, angefangen von der einfachen Gingivitis über die Parodontitis bis hin zur Periimplantitis, infolge der pathogene Noxa in der abnormen Präsenz einiger Bakterienarten und ihrer Stoffwechselprodukte (Biofilm) eine keimtötende oder desinfizierende Wirkung bei der Mundpflege.

Als eine der wichtigsten Waffen im Kampf gegen derartige bakterielle Infektionen sind Antibiotika anzusehen. Diese haben die gesundheitsbezogene Lebensqualität des Menschen seit ihrer Einführung stark verbessert. Diese Vorteile für die Gesundheit sind jedoch in den letzten Jahrzehnten in Gefahr geraten, da viele häufig verwendete Antibiotika gegen bestimmte Krankheiten immer weniger wirksam geworden sind, nicht nur, weil viele von ihnen toxische Reaktionen hervorrufen, sondern auch wegen des Auftretens arzneimittelresistenter Bakterien.

Die Entwicklung von Resistenzen ist mittlerweile ein ernsthaftes Problem, da die bakterielle Resistenz oft nicht auf das spezifische, dem Patienten verschriebene Antibiotikum beschränkt ist, sondern sich im Allgemeinen auch auf andere Verbindungen derselben Klasse erstrecken kann. Die bakterielle Resistenz und ihre rasche Zunahme ist für die globale öffentliche Gesundheit von großer Bedeutung und entwickelt sich zu einer der größten Herausforderungen für die menschliche Gesundheit.

Die Behandlung bakterieller Infektionen mit Antibiotika ist somit nützlich, aber ihr weit verbreiteter Einsatz hat zu alarmierenden Resistenzen bei Mikroorganismen und letztlich zum Wiederauftreten alter Infektionskrankheiten geführt. Daher ist ein auf Antibiotika beruhender Ansatz zur Dezimierung der Keimbelastung in der menschlichen Mundhöhle gerade bei chronischen oder dauerhaften Krankheitsbildern nicht wünschenswert.

Der Erfindung liegt daher die Aufgabe zugrunde, eine Mundspülung anzugeben, die im Hinblick auf den vorgesehenen Einsatz im menschlichen Mund- und Rachenraum bei hoher Verträglichkeit für den Menschen oder menschliches Gewebe eine im Vergleich zu bekannten Mundspülungen deutliche erhöhte keimtötende Wirkung aufweist. Des Weiteren soll eine besonders geeignete Verwendung für die Mundspülung angegeben werden.

Bezüglich der Mundspülung wird diese Aufgabe erfindungsgemäß gelöst, indem sie gebildet ist durch eine wässrige Zusammensetzung, die als Bestandteile zusätzlich zu Wasser eine Anzahl von Pflanzenextrakten und einen antimikrobiellen Wirkstoff aufweist.

Die Erfindung geht dabei von der Überlegung aus, dass die erwünschte keimtötende Wirkung der Mundspülung unter bestmöglichem Verzicht auf Antibiotika und die damit gegebene Gefahr einer Resistenzenbildung erreicht werden kann durch gezielte Bereitstellung eines alternativen Mittels zur Behandlung der Infektionskrankheiten. Als ein solches alternatives Mittel sind Pflanzenextrakte vorgesehen, deren Wirksamkeit gegen resistente Mikroorganismen allein oder in Kombination mit geeigneten Antibiotika ein weites Feld aktueller Forschung darstellt. Der letztgenannte Ansatz, d. h. die Kombinationstherapie oder die synergistische Therapie gegen resistente Mikroorganismen, kann zu neuen Wegen der Behandlung von Infektionskrankheiten führen und stellt wahrscheinlich ein potenzielles Gebiet für weitere Untersuchungen in der Zukunft dar. Die Kombinationstherapie ist insbesondere für Patienten mit schweren Infektionen vorgesehen, die durch arzneimittelresistente Erreger verursacht werden. Die Wirkungsweise der Kombinationstherapie unterscheidet sich erheblich von der der Medikamente selbst, die einzeln wirken; daher ist die Auswahl einer geeigneten Kombination grundlegend und wesentlich und erfordert das Verständnis der möglichen Wechselwirkung zwischen Pflanzenextrakten und antimikrobiellen Wirkstoffen.

In der Kräutermedizin gibt es potenziell bedeutende Vorteile, die mit den synergistischen Interaktionen, die zwischen verschiedenen Antibiotika untereinander oder Pflanzenextrakten bestehen können, oder mit der Synergie zwischen Antibiotikum und Pflanzenextrakt verbunden sind. Die Vorteile sind: (1) erhöhte Effizienz (2) Verringerung der Nebenwirkungen (3) erhöhte Stabilität oder Bioverfügbarkeit freier Wirkstoffe (4) Erzielung einer adäquaten therapeutischen Wirkung bei relativ geringen Dosen im Vergleich zu einem synthetischen Medikament. Es hat sich herausgestellt, dass pflanzliche Antimikroben synergistische Verstärker sind, da sie zwar allein keine antimikrobiellen Eigenschaften haben können, aber bei gleichzeitiger Einnahme mit Standardmedikamenten ihre Wirkung verstärken.

Allerdings sind solche pflanzlichen Wirkstoffe nicht per se für die Behandlung der Mundschelimhaut oder überhaupt für die Anwendung im menschlichen Mund- oder Rachenraum geeignet. Insbesondere weisen derzeit viele auf dem Markt erhältliche Produkte Einschränkungen und in einigen Fällen auch Toxizitätsprofile auf, die einen solchen Einsatz ausschließen. So erfordert beispielsweise die Verwendung ätherischer Öle, dass sie durch chemische Extraktionsverfahren gewonnen werden, bei denen aufgrund der verwendeten chemischen Lösungsmittel häufig toxische Rückstände entstehen. Im Hinblick darauf, dass die Mundschleimhaut über eine eigene Absorption verfügt (siehe sublinguale entzündungshemmende Tabletten, Trinitrin und mehr), ist es nicht wünschenswert, durch Extraktionsverfahren toxische Rückstände in die Mundhöhle einzubringen.

Um demgegenüber die Nutzung pflanzlicher Extrakte auch in der Mundhöhle bei weitgehendem Verzicht auf Antibiotika zu ermöglichen, ist erfindungsgemäß nunmehr die synergistische Kombination mit einem weiteren Bestandteil in der Mundspüllösung, nämlich einem antimikrobiellen Wirkstoff, vorgesehen. Als im Hinblick auf die angestrebte synergistische Wirkung in der Kombination mit Pflanzenextrakten besonders geeignete Basis werden dabei erfindungsgemäß zwei Eckpfeiler der antimikrobiellen und antiviralen Therapie, nämlich in ganz besonders vorteilhafter Ausgestaltung Ethylalkohol und Wasserstoffperoxid, ins Auge gefasst. Diese werden nicht als Antibiotika per se angesehen, und die Resistenzenbildung ist dabei ebenfalls nicht gegeben. Völlig überraschend hat sich herausgestellt, dass diese Grundstoffe, insbesondere in geeignet gewählter Kombination miteinander, spezifisch nicht nur die Wirkung von Kräutern verstärken, sondern auch eine bemerkenswerte Stabilität des Produkts nach der Verpackung bewirken. Vorteilhafte Ausgestaltungen sind Gegenstand der Unteransprüche.

Wie bereits erläutert ist in ganz besonders bevorzugter Ausgestaltung als antimikrobieller Wirkstoff eine Kombination aus Alkohol und Wasserstoffperoxid (H₂O₂) vorgesehen. Die wässrige Zusammensetzung umfasst dabei zusätzlich zu Wasser vorteilhafterweise die Hauptbestandteile (in Gewichts-%) wie folgt:

| | |
|---|---|
| - Alkohol, nämlich Ethylalkohol: | 5-75 % |
| - Wasserstoffperoxid: | 0,025-20 % |
| - ein Pflanzenextrakt: | 0,025-10 % |

Als Pflanzenextrakt sind dabei vorteilhafterweise, einzeln oder in beliebiger Kombination miteinander, die Bestandteile Pfefferminzöl, Eukalyptusöl, Teebaumöl, Niemöl und/oder Rosmarinöl, jeweils mit einem Anteil von 0,05-5 % (in Gewichts-%), vorgesehen.

In ganz besonders bevorzugter Ausgestaltung ist die Mundspülung bzw. die sie bildende wässrige Zusammensetzung zusätzlich zu Wasser aus folgenden Hauptbestandteilen zusammengesetzt (in Gewichts-%):

| | |
|---|---|
| - Alkohol, nämlich Ethylalkohol: | 5-75 % |
| - Wasserstoffperoxid: | 0,025-20 % |
| - Pfefferminzöl: | 0,05-10 % |
| - Eukalyptusöl: | 0,025-5 % |
| - Teebaumöl | 0,025-5 % |
| - Niem-Öl | 0,025-5 % |
| - Rosmarinöl: | 0,05-5 %. |
| - Bergamotte-Öl | 0,025-5 % |

Die Mundspülung kann in der beschriebenen wässrigen Zusammensetzung zur unmittelbaren Verwendung vorgesehen sein. Alternativ kann sie in besonders bevorzugter Ausgestaltung aber auch mit einem geeignet gewählten Gelbildner kombiniert sein, so dass sich mit diesen beiden Hauptbestandteilen eine gelartige Zusammensetzung zur Mundpflege ergibt.

In besonders vorteilhafter und als eigenständig erfinderisch angesehener Ausgestaltung ist die Verwendung einer solchen, vorstehend beschriebenen Mundspülung zur Verminderung der Keimbelastung im menschlichen Mund- oder Rachenraum und/oder zur Zahnpflege vorgesehen.

Die mit der Erfindung erzielten Vorteile bestehen insbesondere darin, dass durch die vorgesehene Kombination eines pflanzlichen Wirkstoffs, insbesondere eines Pflanzenextrakts, mit einem antimikrobiellen Wirkstoff unter Verzicht auf Antibiotika und die dadurch möglicherweise ausgelösten Resistenzbildungen besonders gut verträgliche und gleichermaßen hochwirksame Reduktionen der Keim- oder Bakterienlast in der Mundhöhle erreichbar sind. Wie sich völlig überraschend herausgestellt hat, zeigen dabei gerade die gängigen antimikrobiellen Wirkstoffe Wasserstoffperoxid und Alkohol, in geeigneter Kombination miteinander, synergistische Wirkung mit den pflanzlichen Extrakten, so dass eine besonders wirkungsvolle Reduktion der Keimbelastung und damit einhergehende die Behandlung von Entzündungen ermöglicht ist.

## Patentansprüche

1. Mundspülung, insbesondere zur Behandlung des menschlichen Mund- und Rachenraums oder zur Zahnpflege, gebildet durch eine wässrige Zusammensetzung, die als Bestandteile zusätzlich zu Wasser eine Anzahl von Pflanzenextrakten und einen antimikrobiellen Wirkstoff aufweist.

2. Mundspülung nach Anspruch 1, bei der als antimikrobieller Wirkstoff eine Kombination aus Alkohol und Wasserstoffperoxid (H₂O₂) vorgesehen ist.

3. Mundspülung nach Anspruch 2, deren wässrige Zusammensetzung zusätzlich zu Wasser als Hauptbestandteile aufweist (in Gewichts-%):
| | |
|---|---|
| - Alkohol, nämlich Ethylalkohol: | 5-75 % |
| - Wasserstoffperoxid: | 0,025-20 % |
| - ein Pflanzenextrakt: | 0,025-10 % |

4. Mundspülung nach einem der Ansprüche 1 bis 3, deren wässrige Zusammensetzung als Pflanzenextrakt Pfefferminzöl mit einem Anteil von 0,05 - 10 % (in Gewichts-%) aufweist.

5. Mundspülung nach einem der Ansprüche 1 bis 4, deren wässrige Zusammensetzung als Pflanzenextrakt Eukalyptusöl mit einem Anteil von 0,025 - 5 % (in Gewichts-%) aufweist.

6. Mundspülung nach einem der Ansprüche 1 bis 5, deren wässrige Zusammensetzung als Pflanzenextrakt Teebaumöl mit einem Anteil von 0,025 - 5 % (in Gewichts-%) aufweist.

7. Mundspülung nach einem der Ansprüche 1 bis 6, deren wässrige Zusammensetzung als Pflanzenextrakt Niemöl mit einem Anteil von 0,025 - 5 % (in Gewichts-%) aufweist.

8. Mundspülung nach einem der Ansprüche 1 bis 7, deren wässrige Zusammensetzung als Pflanzenextrakt Rosmarinöl mit einem Anteil von 0,05 - 5 % (in Gewichts-%) aufweist.

9. Mundspülung nach einem der Ansprüche 1 bis 7, deren wässrige Zusammensetzung als Pflanzenextrakt BERGAMOTTE-ÖL mit einem Anteil von 0,05 - 5 % (in Gewichts-%) aufweist.

10. Mundspülung nach einem der Ansprüche 1 bis 9, deren wässrige Zusammensetzung zusätzlich zu Wasser als Hauptbestandteile aufweist (in Gewichts-%):
| | |
|---|---|
| - Alkohol, nämlich Ethylalkohol: | 5-75 % |
| - Wasserstoffperoxid: | 0,025-20 % |
| - Pfefferminzöl: | 0,05-10 % |
| - Eukalyptusöl: | 0,025-5 % |
| - Teebaumöl | 0,025-5 % |
| - Niem-Öl | 0,025-5 % |
| - Rosmarinöl: | 0,05-5 % |
| - Bergamotte-Öl | 0,025-5 % |

11. Gelartige Zusammensetzung zur Mundpflege, die als Hauptbestandteile eine Mundspülung nach einem der Ansprüche 1 bis 9 und einen Gelbildner aufweist.

12. Verwendung einer Mundspülung nach einem der Ansprüche 1 bis 10 zur Verminderung der Keimbelastung im menschlichen Mund- oder Rachenraum.

13. Verwendung einer Mundspülung nach einem der Ansprüche 1 bis 10 zur Zahnpflege.
